# EUROPEAN PATENT SPECIFICATION

(11) **EP 0 732 101 B1**
(45) Date of publication and mention of the grant of the patent: **23.06.1999**
(21) Application number: 96103701.7
(22) Date of filing: 28.03.1991
(51) Int. Cl.: A61K 31/135

(54) **Use of L-deprenyl for retarding age dependent deterioration of the immune system function in mammals**
Verwendung von L-Deprenyl zur Hemmung des Alterniedergangs des Immunsystems in Säugetieren
Utilisation de L-deprenyl pour ralentir la détérioration du système immunitaire due au viellissement chez les mammifères

(30) Priority: 31.08.1990 US 576011; 18.01.1991 US 643452
(43) Date of publication of application: 18.09.1996
(62) Divisional of application: 91302820.5
(73) Proprietor: DEPRENYL ANIMAL HEALTH, INC., Overland Park, Kansas 66210 (US)
(72) Inventor: Milgram, Norton W., Scarborough, Ontario, MIC 1A4 (CA); Stevens, David R., Overland Park Kansas 66213 (US); Ivy, Gwendolyn O., Scarborough, Ontario MIC 1A4 (CA)
(74) Representative: Thomson, Paul Anthony

(56) References cited:
- LIFE SCIENCES, vol. 47, no. 5, 1990, pages 415-420, XP000575064 MILGRAM, N.W. ET AL: "Maintenance on l-deprenyl prolongs life in aged rats"
- ADVANCES IN PHARMACOLOGICAL RESEARCH AND PRACTICE, vol. 3, 1985, pages 7-26, XP000575183 KNOLL, J.: "sTRIATAL DOPAMINE, AGING AND DEPRENYL"
- LIFE SCIENCES, vol. 45, no. 6, 1989, pages 525-531, XP000575063 KNOLL, J.: "Striatal dopamine, sexual activity and lifespan. Longevity of rats treated with (-)deprenyl"
- PHARMACOLOGICAL RESEARCH COMMUNICATIONS, vol. 20, no. IV, 1988, pages 111-112, XP000574691 BALSA, M.D. ET AL: "Kinetics of inhibition of pig leukocyte MAO by the acetylenic inhibitors l-deprenyl and pargyline"
- MECHANISMS OF AGING AND DEVELOPMENT, vol. 36, no. 3, 1986, pages 223-241, XP000575077 NEGORO, S. ET AL: "Mechanisms of age-related decline in antigen-specific T cell proliferative response: IL-2 induced proliferative response of purified TAC-positive T cells"
- JOURNAL OF CLINICAL INVESTIGATION, vol. 67, no. 4, 1981, pages 937-42, XP002007959 GILLIS, S. ET AL: "Immunological studies of aging"
- CELLULAR IMMUNOLOGY, vol. 63, no. 1, September 1981, pages 16-27, XP000575113 MORGAN, E.L. ET AL: "The immune response in aged 57BL/6 mice"

## Description

L-deprenyl is a selective monoamine oxidase B (MAO-B) inhibitor, which is widely used as an adjunct in the treatment of Parkinson's disease. While its most cordon usage is for the treatment of Parkinson's disease, L-deprenyl was originally developed as an antidepressant agent. Recent testing has indicated that L-deprenyl may have some effect on increasing sexual response in aging animals, and also may have some effect, at least in rats in increasing the natural life span. However, to date L-deprenyl has only been medically approved by regulatory agencies for use as an adjunctive treatment for Parkinson's disease.

The search for new lines of medication to improve the quality of life in senescence ever continues. This becomes especially important in modern-day society, especially in developed countries, where the proportion of citizens over 65 years of age continues to increase. In sum, the quality of life has become increasingly important in older years, as people continue to experience longer life expectancy.

There is, therefore, a continuing and real need for the development of medications which retard the normal deterioration of certain bodily functions.

Accordingly the present invention relates to the use of L-deprenyl to retard deterioration of cognitive abilities during advanced aging, and to retard the deterioration in the natural tendency for curious or exploratory behaviour in aged mammals, such as pets like dogs and cats.

Also of particular importance to the present invention are the physiological functions of the normal immune system in aging mammals. The physiologic effects of L-deprenyl may be wide ranging and may impact additional bodily functions other than those discussed in this case and its parent application. Examples may include salivary gland secretion, or other exocrine gland secretions such as pancreas associated with the G.I. tract. Another example might be retention of cardiovascular function or retention of normal wound healing capability.

Thus, the present invention is concerned with the use of L-deprenyl for retardating the age-dependent deterioration of normal immune system function in mammals, providing that the doses hereinafter discussed are used.

While L-deprenyl is a known compound, it has never before been used at an level for maintenance of the immune system.

Like most drugs, L-deprenyl can have diverse physiological effects which are completely dependent upon the dose administered. In accordance with the present invention, L-deprenyl can be used for successful methods of treatment to provide the desired physiological effects enumerated herein, providing that it is used at the dosage levels mentioned herein, and providing it is administered at the periodic intervals and for the length of time mentioned herein. Obviously, when different dosages and levels of treatment are used, the results expressed herein may not be achieved. In fact, at higher doses, adverse behavioural effects may be encountered.

The method and means of accomplishing this as well as other primary objectives of the present invention will be apparent from the detailed description which will follow hereinafter.

The present invention relates to the process of using a known compound, L-deprenyl, for a new use. In particular, at the dosage levels described herein, providing that the dosage is used for at least the periods of time expressed herein, there is an observed maintenance of normal immune function for longer periods of time in aging mammals. The treatment is specially useful for domesticated pets like dogs and cats, as they increase in age, but would be expected to have utility in any mammalian species, including humans.

### DETAILED DESCRIPTION OF THE INVENTION

As earlier stated, the compound that is useful for the method or protocol of the present invention is a known compound, L-deprenyl. L-deprenyl has the formula (-)-N-α-dimethyl-N-2-propynylbenzene-ethanamine. It can be illustrated by the following graphic formula:

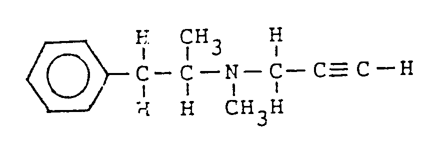

L-Deprenyl also is at times referred to as (-)deprenyl to illustrate that it is a levorotary isomer which is the active form for treatment of Parkinson's disease. Typically, it is provided in a pharmaceutically acceptable salt form thereof such as the hydrochloride salt.

As used here, pharmaceutically acceptable salt form thereof, means the following. Acceptable for use in the pharmaceutical or veterinary art, being nontoxic or otherwise not pharmaceutically or veterinary unacceptable. "Acceptable salt form thereof" means salts formed with inorganic acids such as hydrochloric acid, hydrobromic acid, sulfuric acid, nitric acid, phosphoric acid, and the like, and as well organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, etc.

Administration of the prophylactively active compound L-deprenyl to achieve physiological results of the present invention can be via any of the accepted modes of administration for systemically active substances. These methods include oral, parenteral, and otherwise systemic, aerosol, and topical forms, as well as sustained release systems, etc.

The compositions of the present invention may be any of those known in the pharmaceutical and veterinary arts which are suitable for the method of administration and dosage required in any particular circumstance. In the case of both pharmaceutical and veterinary applications, such compositions may include tablets, pills, capsules, powders, aerosols, suppositories, skin patches, parenterals, and oral liquids including oil aqueous suspensions, solutions and emulsions. It may include long acting injectables and sustained release devices.

When the dosage is in solid form, solid pharmaceutical carriers such as starch, sugar, talc, mannitol, povidone, magnesium stearate, and the like may be used to form powders. Lactose and mannose are the preferred solid carrier. The powders may be used as such for direct administration to a patient or, instead, the powders may be added to suitable foods and liquids, including water, to facilitate administration.

The powders also may be used to make tablets, or to fill gelatin capsules. Suitable lubricants like magnesium stearate, binders such as gelatin, and disintegrating agents like sodium carbonate in combination with citric acid may be used to form the tablets.

Unit dosage forms such as tablets and capsules may contain any suitable predetermined amount of L-deprenyl, advisably as a nontoxic acid addition salt, and may be administered one or more at a time at regular intervals as later described. Such unit dosage forms, however, should with a broad range guideline contain a concentration of 0.1 mg/kg to 5.0 mg/kg of one or more forms of the active L-deprenyl.

A typical tablet for the specified use mentioned herein in a 25 kg dog may have the composition:

| | Mg. |
|---|---|
| 1. L-deprenyl | 25 |
| 2. Mannitol | 100 |
| 3. Stearic acid | 3 |

A granulation is made from the mannitol. The other ingredients are added to the dry granulation and then the tablets are punched.

Another tablet may have the composition:

| | Mg. |
|---|---|
| 1. L-deprenyl | 25 |
| 2. Starch U.S.P. | 57 |
| 3. Lactose U.S.P. | 73 |
| 4. Talc U.S.P. | 9 |
| 5. Stearic acid | 6 |

Powders 1, 2 and 3 are slugged, then granulated, mixed with 4 and 5, and tableted.

Capsules may be prepared by filling No. 3 hard gelatin capsules with the following ingredients, thoroughly mixed:

| | Mg. |
|---|---|
| 1. L-deprenyl | 25 |
| 2. Lactose U.S.P. | 200 |
| 3. Starch U.S.P. | 16 |
| 4. Talc U.S.P. | 8 |

The following comments refer to various uses of L-deprenyl, or a pharmaceutically acceptable form thereof. It is to be understood that the use of such compound for the manufacture of a medicament for retarding the age-dependent deterioration of immune system function in mammals is in accordance with the present invention. The other uses described are not in accordance with the invention and are merely supplied by way of information.

Aging has long been thought of as being associated with decreased adaptiveness to stress (Selye and Tuchweber, "Stress in relation to aging and disease", Hypothalamus, Pituitary and Aging, pp. 557-573, 1976). For example, basal body temperature is similar in both young and aged humans, but aged individuals are less able to regulate their temperature when heat or cold challenged (Shock, "Systems integration", In: Handbook of the Biology of Aging, New York, 1977). There is also some evidence that stress itself can accelerate certain biomarkers of aging (Curtis, "Biological mechanisms underlying the aging process", Science 141, 686, 1963; Pare, "The effect of chronic environmental stress on premature aging in the rat", J. Gerontol, 20, 78, 1965). Studies by Sapolsky's and Landfield's groups support the notion of a "glucocorticoid cascade" (Sapolsky et al., "The neuroendocrinology of stress and aging: The glucocorticoid cascade hypothesis", Endocrine Reviews 7, 284-301, 1986), in which both aging and stress lead to a decreased ability to terminate the secretion of adrenocortical stress hormones (glucocorticoids) at the end of a stress response. Cumulative exposure to glucocorticoids causes degenerative changes in the hippocampus, a brain region which normally inhibits glucocorticoid release. The degeneration, in turn, further decreases the brain's ability to terminate the stress response, thus leading to more damage and effectively forming a feed-forward cascade with potentially serious pathophysiological consequences in the old individual. For example, an overabundance of glucocorticoids, as seen in prolonged stress and in Cushing's syndrome, can cause myopathy, steroid diabetes, hypertension, immunosuppression, infertility and inhibition of growth (Munck et al., "Physiological functions of glucocorticoids during stress and their relation to pharmacological actions", Endocrine Review 5, 25-49, 1984; Krieger, "Cushing's syndrome", Monographs on Endocrinology 22, 1-122, 1982).

Although the thyroid gland may have sufficient capacity to maintain its principal hormones, T3 & T4, within normal ranges during the senescent phase of life, there is some suggestion that age-related intermittent thyroid deficiency may arise as a consequence of stress, or that the thyroid hormones are potentially less biologically effective (Morse, D. R. Aging: Causes and Control. Internat. J. Psychosomat. 35: 12-42, 1988). Likewise, thyroid stimulating hormone (TSH), secreted from the pituitary gland, functions within the negative feedback control mechanism associated with the hypothalamus, portions of which operate under the trophic influences of the dopaminergic neurons. With age, the nigrostriatal dopamine secreting neurons degenerate, with the possible consequence of perturbation of the hypothalamic-hypophyseal system, again predicting a decrease of thyroid gland control during senescence. Further, hypothyroidism is often associated with aberrant immune function, often termed immune-mediated or autoimmune hypothyroidism. This, too, may be a consequence of aging (see below).

Immune reactions involve the coordinated efforts of three lymphocyte subpopulations: T and B lymphocytes and antigen presenting cells. Substantial evidence exists (mainly in humans and mice) that immune function declines with age and that impaired T-cell function may be largely responsible for the decline. While some changes in B lymphocyte function have been reported, it is difficult to dissociate these effects from concomittant changes in T-Cells. Also, little or no evidence supports changes in antigen presenting cells with age.

On the other hand, a number of investigators have shown a substantial decline in most measures of T-cell function with age. The most studied of the immune parameters is lymphocyte proliferation. The proliferative capacity of both T- and B-cells has been found to decline substantially with age (Morgan et al., "The immune response in aged C57BL/6 mice. I. Assessment of lesions in the B-cell and T-cell compartments of aged mice utilizing the Fc fragment-mediated polyclonal antibody response", Cellular Immunology 63, 16-27, 1981; Abraham et al., "Reduced in vitro response to concanavalin A and lipopolysaccharide in senescent mice: A function of reduced number of responding cells", European Journal of Immunology 7, 301-304, 1977; Hefton et al., "Immunologic studies of aging. V. Impaired proliferation of PHA responsive human lymphocytes in culture", Journal of Immunology 125, 1007-1010, 1980). These studies utilize splenocytes which are mitogenically challenged with phytohemaglutin (PHA) or concanavalin-A in vitro in the presence of 3H-thymidine or bromodeoxyuridine (BrdU). Thymidine or BrdU uptake by the cells is then measured and reflects the number of cells entering mitosis. Also, upon activation by mitogens or antigens, T-cells secrete a number of antigen-non-specific growth and maturation factors, collectively termed lymphokines. One of these, Interleukin-2 (IL-2), seems to be required for T-cell division and plays a role in B-cell growth as well. T-cells from old mice and humans have been found to secrete diminished amounts of IL-2 when triggered by mitogens, alloantigens or foreign antigens (Gillis et al., "Immunological studies of aging. Decreased production of and response to T cell growth factor by lymphocytes from aged humans", Journal of Clinical Investigation 67, 937-942, 1981; Miller and Stutman, "Decline, in aging mice, of the anti-TNP cytotoxic T cell response attributable to loss of Lyt-2, IL-2 producing helper cell function", European Journal of Immunology 11, 751-756, 1981; Nagel et al., "Decreased proliferation interleukin 2 synthesis, and interleukin 2 receptor expression are accompanied by decreased mRNA expression in phytohemag-glutinin-stimulated cells from elderly donors", Journal of Clinical Investigation 81, 1096-1102, 1988; Thoman and Weigle, "Partial restoration of Con A-induced proliferation, IL-2 receptor expression, and IL-2 synthesis in aged murine lymphocytes by phorbol myristate acetate and ionomycin", Cellular Immunology 114, 1-11, 1988). Further, several studies have shown that aging leads not only to poor production of IL-2, but also to diminished responsiveness to this growth factor. Thus, addition of exogenous IL-2 leads to only a partial restoration of function in immune cells from old animals (Gillis et al., "Immunological studies of aging. Decreased production of and response to T cell growth factor by lymphocytes from aged humans", Journal of Clinical Investigation 67, 937-942, 1981; Gilman et al., "T lymphocytes of young and aged rats. II. Functional defects and the role of interleukin-2", Journal of Immunology 128, 644-650, 1982; Gottosman et al., "Proliferative and cytotoxic immune functions in aging mice. III. Exogenous interleukin-2 rich supernatant only partially restores alloreactivity in vitro", Mechanisms of Ageing and Development 31, 103-113, 1985). Also, the number of T-cells able to express IL-2 receptors upon stimulation by a mitogenic agent declines with age in both humans and mice (Negoro et al., "Mechanisms of age-related decline in antigenspecific T cell proliferative response: IL-2 receptor expression and recombinant IL-2 induced proliferative response of purified TAC-positive T cells", Mechanisms of Ageing and Development 36, 223-241, 1986; Vie and Miller, "Decline, with age, in the proportion of mouse T cells that express IL-2 receptors after mitogen stimulation", Mechanisms of Ageing and Development 33, 313-322, 1986). Aging, therefore, leads to a decline in both production of and response to IL-2.

Growth hormone (GH) levels are known to fall in both male and female rats with age. In female rats, a 50% decrease in GH secretion can be demonstrated at 11 months and may account for the stasis in body growth which occurs at about this time (Takahasi, S., Goya, R., and Meites, J., "Growth hormone secretory patterns in young, middle aged and old female rats", Neuroendocrinology 46, 137-142, 1987). Aging male rats show a marked decrease in GH secretion, but the time course of the decrease is not known (Sonntag et al., "Decreased pulsatile release of growth hormone in old male rats", Endocrinology 107, 1875-1879, 1980). Somatomedin (SM) levels in the circulation, which are under GH control, also fall markedly with age. These decreases in GH and SM secretion are of primary importance as they may cause the age related decrease in protein synthesis, which may have far reaching implication for the ability of aged organisms to maintain body composition and homeostasis.

The total amount of protein in the human body decreases with age after about 40 years, due largely to a loss of skeletal muscle mass (Young et al., "Human aging: protein and amino acid requirements", Nutritional Approaches to Aging Research, 47, CRC Press, 1982). This decrease is likely due to decreases in protein synthesis which are known to occur in a variety of organisms with age. Body water also decreases with age in humans, beginning at birth and proceeding at a fairly constant rate throughout life (Shock et al., "Age differences in the water content of the body as related to basal oxygen consumption in males", J. Gerontol 18, 1, 1963; Timiras, Developmental Physiology and Aging, Macmillan, New York, 1972). Total body fat increases with age in humans, until at least the fifth decade of life; data on humans of advanced age are conflicting (Meneely et al., "Analysis of factors affecting body composition determined from potassium content in 915 normal subjects", Ann. N.Y. Acad. Sci. 110, 271, 1963; Forbes and Reina, "Adult lean body mass with age: some longitudinal observations", Metabolism 19, 653, 1970; Myhre and Kessler, "Body density and potassium 40 measurements of body composition as related to age", J. Appl. Physiol. 21, 1251, 1966; Novak, "Aging, total body potassium, fat free mass in males and females between the ages of 18 and 85 years", J. Gerontol 27, 438, 1972). In contrast, in Fischer 344 rats, total body fat increases until about 75% of their lifespan, but decreases after that (Bertrand et al., "Changes in adipose mass and cellularity throughout the adult life of rats fed ad libitum or a life-prolonging restricted diet", J. Gerontol. 35, 827, 1980). Specific age related changes in serum lipids are difficult to establish because of their dependence on whole body fat, exercise and diet, and available data are conflicting. Similarly, changes in serum lipoprotein levels with age are influenced by a number of factors and do not appear to be age- so much as health and habit-related.

Serum albumin levels in humans appear to be unaffected by age. In rats, albumin levels appear to depend on strain and on whether chronic nephropathy is common, as in Fischer 344 rats, which have nephropathy and concomittant decreases in serum albumin levels (Heiss et al., "The epidemiology of plasma HDL-cholesterol levels: The Lipid Research Clinic Prevalence Study, Summary", Circulation 62 (Suppl. 4), 116, 1980). Connective tissue changes are common with age. Collagen, the major protein component, becomes more insoluble, rigid, and resistant to enzyme digestion with age (Hamlin and Kohn, "Evidence for progressive age-related structural changes in post-mature human collagen", Biochim. Biophys. Acta 236, 458, 1971; Everitt and Delbridge, "Two faces of collagen ageing in the tail tendon of hypophysectomized rats", Exp. Gerontol. 7, 45, 1972). However, in young and aging male and female Wistar rats, no changes in collagen were found with age (Porta et al., "Effects of the type of dietary fat at two levels of vitamin E in Wistar male rats during development and aging. II. Biochemical and morphometric parameters of the brain", Mech. Ageing Dev. 13, 319, 1980; "Effects of the type of dietary fat at two levels of vitamin E in Wistar male rats during development and aging. III. Biochemical and morphometric parameters of the liver", Mech. Ageing Dev. 15, 297, 1981; "Effects of the type of dietary fat at two levels of vitamin E in Wistar male rats during development and aging. IV. Biochemical and morphometric parameters of the heart", Mech. Ageing Dev. 18, 159, 1982). Thus, reliable age-related changes in body composition across species appear to be restricted to decreases in total body protein and water content.

From the above descriptions, it can be seen that age can adversely impact each of the functions of the adrenal gland, the thyroid, the immune system and body composition as mammals get older. Here, providing that the dosages hereinafter described are used, these functions can be maintained for longer periods of time in older animals at normal levels.

The term "mammal" as used herein includes without limitation humans and domesticated animals such as cattle, horses, swine, sheep, dogs, cats, goats and the like. The tests hereinafter shown in the examples are particularly illustrative for dogs, particularly beagles, but indicate usage for other domesticated pets including cats. The treatment may even work for birds or fish.

Needless to say, the natural enjoyment of these pets would be significantly increased in their older age if one could retard the decrease in the enumerated physiological functions. These natural changes alter the animal's personality such that the human owner has less enjoyment from the animal.

In accordance with the present invention, it is illustrated that each of the above enumerated physiological functions can be maintained for longer periods if the animal is treated periodically with small but prophylactically effective doses of L-deprenyl.

As hereinafter explained, the dosage regimen to achieve these desirable results differs considerably from the dosage regimen used in treating Parkinson's disease. At the highest doses recommended for uses disclosed herein, the levels are less than one-half the amount used for treating Parkinson's disease. In particular, the dosage regimen of the present invention shows usage at levels from about 0.1 mg/kg of body weight up to about 5.0 mg/kg of body weight from 1 to 5 times weekly, but preferably on alternate days. Most preferably the dosage level is 1-2 mg/kg of body weight given twice weekly, starting in mid-life. Of course it would be known to those in the art that sustained release systems can be used to provide less frequent administration to achieve the required dosage level.

It is not known precisely why the use of L-deprenyl at the dosage levels and periodicity expressed herein achieves these results. While not wishing to be bound by any theory of operation of the present process, it is believed that rather than solely affecting brain chemistry, there may be a direct effect on the organs involved. The effect may be hypotensive. As explained in the examples below, analysis of serum chemistry perhaps suggests that the L-deprenyl treatment may have a direct effect on specific organ function. With regard to the maintenance of body composition, it is simply not known by what mechanism the compound works, except to say that it is critically important that the dosage be at levels expressed herein rather than at Parkinson's disease levels, otherwise adverse effects may be achieved, particularly in dogs.

In the tests and examples reported hereinafter, differences in renal function between control and 1-deprenyl treated animals was measured by an examination of blood chemistry and in particular the level of blood urea nitrogen (BUN), which is a measure of waste product which is cleared from the body by the kidneys. High levels are indicative of ineffective renal function, and low levels are correspondingly an indication of proper renal function.

Tests of cognitive ability, i.e. memory and response acquisition, were measured in a water maze test. The particular animals were placed in a water maze, as described below and given 90 seconds to swim and locate a platform and climb upon it and escape from the water, for which these animals show a natural aversion. Repeat testing continued until the animals learned that once placed in the water they could swim to the platform and easily prevent drowning. Memory was measured by the ability of the rat to retain this learned behavior five days following the last maze test. In particular, five days after the maze test, the rats were placed in the water maze again, with the platform removed. As a retention measure, time spent in the region of the maze where the platform would normally have been was recorded. The same procedure was repeated twice more each time with the platform in a new location. By doing so, the animals' ability to profit from its previous experience was tested.

During the late stages of aging, rats show a progressive decrease in body mass, and an examination of individual data indicates that decreased body weights proved to be a reliable predictor of death. In the test data of the rats studied in the examples, body weights of the controls and the 1-deprenyl treated rats were recorded. It was uniformly observed that normal age dependent body mass loss was retarded in the 1-deprenyl treated group and that this was not associated with dietary differences. In sum, at advanced stages of aging, the surviving 1-deprenyl rats were significantly heavier than the surviving controls.

In the following examples, those illustrating retarding the age-dependent deterioration of immune system function in mammals are in accordance with the invention, the remaining examples being given by way of information and not being in accordance with the invention.

### EXAMPLES

In the examples below, male Fischer rats from Harlan Sprague Dawley were used. The animals were obtained at 21-23 months of age. They were allowed free access to food and water, and were weighed on every other day.

L-deprenyl treatment was started when the animals were between 24 and 25 months of age. The drug was administered subcutaneously at a dose of 0.25 mg/kg on alternate days until the animal either died or was removed from the experiments. The deprenyl was dissolved in a solution of saline.

Serum analysis was done on the animals on two occasions, at the start of the experiment, and again after 3 months of treatment. The blood samples were taken intraorbitally, which could only be done on anesthetized animals. As an anesthetic, a mixture of equivalent volumes of ketamine and atravin were used because the combination has a low toxicity, and short duration of effect in young rats. In the baseline test, a dose of .2 ml was used. However, this induced a much deeper and longer lasting period of anesthesia than was desired and the dosage was subsequently reduced to .08 ml for the three month test.

The samples were immediately sent to Vita-Tech Canada, where biochemical assays were done for: glucose, creatinine, bilirubin (total), blood urea nitrogen (BUN), SGOT, SGPT, total protein and albumin. For analysis of hematology, measurements were taken of hemoglobin, hematocrit, RBC counts, WBC counts, MCH, neutrophils, lymphocytes, monocytes, eosinophils, Basophils, and nRBC. A double blind procedure was followed in collection and analysis of serum biochemistry and hematology. The results of the serum analysis are summarized in Table 1. The only significant difference between the controls and L-deprenyl group at three months was in the measure of (BUN). The comparisons for creatinine (p=.129), albumin (p=.117) and A/G ration (p=.151) were close to the significance level, while there were clearly no differences in bilirubin (p=.826), SGOT (p=.990) and SGPT (p=.667). Table 1 also shows the correlation between each of the measures and survival. The levels of BUN, SGOT and bilirubin all correlated significantly.

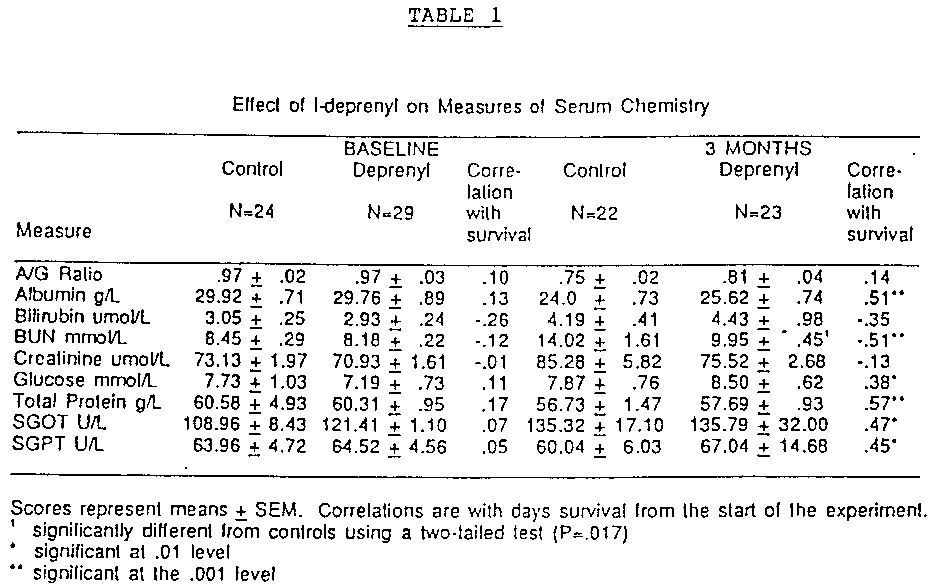

The blood chemistry data were informative, and provide a possible explanation as to why animals treated with L-deprenyl survived longer than the controls. At 26 months, there was a significant difference in the measure of BUN, with the deprenyl group having a lower score than the controls. BUN is a measure of a waste product which is cleared from the body by the kidneys. High levels are therefore indicative of ineffective renal function. Differences in renal function between the deprenyl group and the control group were also indicated by the differences in amounts of creatinine (deprenyl lower than controls), although the size of the differences did not achieve the previously determined level of statistical significance.

In a comparison between 23 and 26 month animals, it was found that both BUN and creatinine were significantly higher in the 26 month test. These results were not unexpected, since it is known that the rat, like other mammals, shows marked deterioration of renal function with advanced age. The significant drug effect at 26 months therefore is indicative of L-deprenyl treatment providing protection of renal function. That such protection is associated with survival is further indicated by the significant correlation between BUN measure and days of survival in the 26 month group.

The absence of differences between the deprenyl and control groups on the other biochemical measures may be indicative of deprenyl affecting the kidneys to a greater extent than it does other peripheral organs, or that the rats were too old at the start of treatment, or that dosages were insufficiently low, or that L-deprenyl does not affect the parameters tested.

Cognitive abilities were measured in the following described manner. The water maze consisted of a circular chamber filled with water to a depth which just covered the surface of a transparent platform, 17.75 cm in height. At the start of each session, the rat was placed in the tank facing the outer surface at a randomly determined point, approximately 1 meter from the center of the platform. Animals were given 90 seconds to locate and climb upon the platform. The animal was removed from the platform after 30 seconds. A correction procedure was used. If the animal did not find the platform, it was gently guided to it at the end of the trial, and removed after 30 seconds. Testing continued until either the criterion of escaping onto the platform within 25 seconds on four out of five consecutive trials was achieved or 30 trials had been completed. A maximum of ten trials were given each day, with an interval of 30 minutes between each trial. Every trial was also videotaped.

Retention was tested five days following the last acquisition trial by placing the rats in the maze for three 90 second trials with the platform removed. As a retention measure, the time was recorded that was spent in the region of the maze where the platform had earlier been located. A second set of acquisition trials were then given using same procedures followed during the original training, except that the platform was moved to another location. Following acquisition and subsequent retention trials, the animals received a third set of trials -- again with the platform at another position. Thus, each animal was tested for both acquisition and retention on three separate occasions.

Changes in cognitive abilities of animals treated with L-deprenyl and control were measured in the water maze test, earlier described.

Water maze acquisition was tested in 16 animals (8 deprenyl and 8 control) at 26 months. The deprenyl group were tested while in their third month of drug treatment. An additional three animals started the testing, but were dropped because of inability to swim acceptably. At 29 months, 9 animals (5 deprenyl and 4 controls) were tested on the water maze. The deprenyl animals were in their sixth month of drug treatment. Five additional rats were unable to swim or were moribund at the start of testing.

Analysis of water maze acquisition was based upon the number of trials required to reach criterion. A maximum score of 33 was assigned if an animal did not learn the task within the three day test period. The results are summarized in Table 2. An analysis of variance with age and session as main effects indicated a significant age effect reflecting slower learning in the older animals (F(2,58)=10.5, p=.00), and a significant session effect reflected improvement in learning over repeated testing (F(2,58)=11.12, p=.00). There was also a tendency for the younger animals to show greater improvement, although the age by session interaction was not significant (F(6,58)=1.64, p=.152).

To test for drug effect in acquisition, the 26 and 29 month animals were compared with a three way analysis of variance with age, group and session as main effects. There were significant main effects for age (F(1,21)=4.62, p=.143) and session (F(2,42)=2.55, p=0.29). The group effect was not significant, the age by group interaction was highly significant (F(2,42)=12.2, p=.002). As indicated in Table 2, this finding is attributable to the deprenyl group performing poorly when tested at 26 months, and better than the controls when tested at 28-29 months.

The results of the retention tests, on the other hand, did not reveal any consistent effects of group or age.

**TABLE 2**

| Mean trials to criterion (± SE) in water maze as a function of age, treatment with L-deprenyl and previous trials | | | | | |
|---|---|---|---|---|---|
| Age (month) | Group | N | Test 1 | Test 2 | Test 3 |
| 7 | Control | 9 | 15.67 ± 1.91 | 10.67 ± 3.18 | 9.33 ± 2.03 |
| 23 | Control | 12 | 18.50 ± 2.49 | 12.25 ± 2.83 | 5.67 ± .86 |
| 26 | Control | 8 | 17.88 ± 3.86 | 15.87 ± 1.87 | 8.00 ± .96 |
| | Deprenyl | 8 | 22.63 ± 3.54 | 22.38 ± 3.85 | 21.75 ± 3.58 |
| 29 | Control | 4 | 31.00 ± 0.71 | 26.25 ± 5.81 | 29.00 ± 4.67 |
| | Deprenyl | 4 | 25.40 ± 2.69 | 17.20 ± 4.22 | 13.80 ± 5.07 |

The control rats showed better acquisition at 26 months than the deprenyl group, while at 29 months the results were reversed - with the deprenyl group showing superior learning. The poor acquisition seen in the 26 month deprenyl-treated rats was largely due to difficulties in reversal -- that is, in relearning the task after the position of the platform was changed. At 29 months, the deficits in the control group reflected a general retardation, indicated by an inability to solve the problem, and are indicative of severe cognitive impairment.

There is no obvious explanation for the results from the 26 month group, but the possibility cannot be ruled out that deprenyl produces a transient cognitive disruption. Very likely some period of time is required on the drug before the animals fully adjust to it. It could, however, be concluded from this data that the deficits were not permanent. To the contrary, the 29 month old animals on deprenyl showed significantly better learning than the controls.

When compared with saline-treated controls, the L-deprenyl treated rats survived longer, appeared healthier, showed more exploratory behavior, and less evidence of mental retardation at 29 months. Additionally, they did not lose as much body weight, which was not due to differences in food consumption.

The following examples, generally prophetic in nature, are presented to provide the illustrative evidence of the likely results for testing with beagles for maintenance of normal thyroid function, normal adrenal function, normal immune system function, and for normal body composition.

Male and female beagles are treated with L-deprenyl or vehicle beginning at four to seven years of age (GH) (SM) and protein synthesis levels are measured two to five years later. L-deprenyl treated dogs are found to maintain significantly higher levels of each of these than do control dogs, thus appearing more like younger dogs. The dose level is 0.1 to 5 mg/kg for a time of months to years, with dosing being from a composition comprising tablets given orally. The frequency of dosing is from one to five times weekly. This example relates to maintenance of body composition.

Male and female beagles are treated with L-deprenyl or vehicle beginning at four to seven years of age. Two to five years later, animals are evaluated for ability to terminate the stress response to 1) prolonged loud noise, 2) cold exposure and 3) immobilization, by measurement of induced vs basal levels of corticosterone in the bloodstream over the subsequent 24 hours. The L-deprenyl treated beagles are significantly more successful in terminating the secretion of corticosterone than are control dogs, thus returning to basal levels of circulating glucocorticoids more quickly. The dogs are subsequently sacrificed and numbers of pyramidal cells in region CA1 of hippocampus, a region especially vulnerable to aging and stress, are quantified. L-deprenyl treated dogs will maintain significantly higher numbers of these neurons than do controls, indicating less age- or stress-induced degeneration in this cognitively important brain region. The dose level is 0.1 mg/kg to 5 mg/kg for a time of months to years, with dosing being from a composition comprising tablets, and given orally. The same frequency of dosing of from one to five times weekly applies here as well as to the treatments later discussed. This test relates to adrenal function.

By slowing the aging process, L-deprenyl will also slow the decline in immune function as determined in several ways. Beagles four to seven years of age are treated with L-deprenyl or vehicle for two to five years. Splenocytes from the dogs are mitogenically challenged and proliferative capacity of the cells are measured. Cells from the L-deprenyl treated dogs display significantly higher levels of proliferation than do cells of control dogs. Further, cells from L-deprenyl treated dogs display both an increase in ability to proliferate in response to IL2 and an increase in secretion of IL2 in response to various antigens. Also, the proportions of T-cells capable of expressing IL2 receptors in response to antigen are significantly higher in L-deprenyl treated than in control dogs. The dose level is 0.1 mg/kg to 5 mg/kg for a time of months to years, with dosing being from a composition comprising tablets, and given orally. This test relates to immune system.

Male and female beagles are administered L-deprenyl beginning at four to seven years of age. Two to five years later, T3 and T4 levels are found to be higher in treated and control males and females. As well, beagles of both sexes are found to have maintained higher levels of TRF, than are control dogs thus approximating T3 and T4 levels of younger beagles. The dose level is 0.1 mg/kg to 5 mg/kg for a time of months to years with dosing being from a composition comprising tablets, and given orally. This test pertains to maintenance of thyroid functions.

It can be seen from the above examples that L-deprenyl when dosed as described herein is effective for use in mammals to maintain normal immune system function.

## Claims

1. The use of the compound L-deprenyl, or a pharmaceutically acceptable form thereof, for manufacture of a medicament for retarding the age-dependent deterioration of immune system function in mammals.

2. The use of the compound as claimed in claim 1, characterised in that the L-deprenyl is the levorotary optical isomer.

3. The use of the compound as claimed in claim 1 or 2, characterised in that the L-deprenyl is the hydrochloride addition salt form thereof.

4. The use of the compound as claimed in claim 1, 2 or 3, characterised in that the medicament is used for a dog.

5. The use of the compound as claimed in claim 1, 2 or 3, characterised in that the medicament is used for a cat.

## Patentansprüche

1. Verwendung der Verbindung L-Deprenyl oder einer pharmazeutisch annehmbaren Form derselben, für die Herstellung eines Medikaments zur Verzögerung der altersabhängigen Zerstörung der Immunsystemfunktion bei Säugetieren.

2. Verwendung der Verbindung gemäß Anspruch 1, dadurch gekennzeichnet, daß das L-Deprenyl das links rotierende optische Isomere ist.

3. Verwendung einer Verbindung gemäß Anspruch 1 oder 2, dadurch gekennzeichnet, daß das L-Deprenyl das Hydrochlorid-Additionssalz davon ist.

4. Verwendung einer Verbindung gemäß Anspruch 1, 2 oder 3, dadurch gekennzeichnet, daß das Medikament für einen Hund verwendet wird.

5. Verwendung einer Verbindung gemäß Anspruch 1, 2 oder 3, dadurch gekennzeichnet, daß das Medikament für eine Katze verwendet wird.

## Revendications

1. Utilisation du composé L-déprényl, ou d'une forme pharmaceutiquement acceptable de celui-ci, pour préparer un médicament pour retarder la dégradation fonction de l'âge du fonctionnement du système immunitaire chez les mammifères.

2. Utilisation du composé selon la revendication 1, caractérisée en ce que le L-déprényl est l'isomère optique lévogyre.

3. Utilisation du composé selon la revendication 1 ou la revendication 2, caractérisée en ce que le L-déprényl se présente sous sa forme de sel d'addition, chlorhydrate.

4. Utilisation du composé selon la revendication 2 ou la revendication 3, caractérisée en ce que le médicament est utilisé pour un chien.

5. Utilisation du composé selon la revendication 2 ou la revendication 3, caractérisée en ce que le médicament est utilisé pour un chat.
